# EUROPEAN PATENT SPECIFICATION

(11) **EP 1 541 155 B1**
(45) Date of publication and mention of the grant of the patent: **26.05.2010**
(21) Application number: 03741220.2
(22) Date of filing: 04.07.2003
(51) Int. Cl.: A61K 33/10, A61K 8/04, A61Q 19/08, A61Q 19/00, A61P 17/00, A61P 17/02

(54) **Material for formation of carbon dioxide external preparation**
Material zur Bildung des topischen Kohlendioxid-Präparats
Substance destinée à la formation d'une préparation à usage externe à base de dioxyde de carbone

(30) Priority: 05.07.2002 JP 2002197622
(43) Date of publication of application: 15.06.2005
(73) Proprietor: Neochemir, Inc., Kobe-shi, Hyogo 651-0087 (JP)
(72) Inventor: TANAKA, Masaya, Kobe-shi, Hyogo 654-0036 (JP)
(74) Representative: Sajda, Wolf E.
(86) International application number: PCT/JP2003/008568
(87) International publication number: WO 2004/004745

(56) References cited:
- EP-A1- 1 386 613
- EP-A1- 1 470 820
- EP-A2- 0 380 253
- WO-A1-99/24043
- JP-A- 2000 319 187

## Description

### TECHNICAL FIELD

The present invention relates to a material for formation of carbon dioxide external preparation in order to prepare a carbon external preparation, by which cosmetic and medical effects are obtained.

### BACKGROUND ART

It is disclosed in Japanese Patent Application Laid-open No. 2000-319187 that compositions for transdermal or transmucosal absorption of carbon dioxide, that are viscous compositions containing carbon dioxide in the form of bubbles, are effective against mucocutaneous damage such as itching, bedsores, wounds, burns, angular stomatitis, stomatitis, skin ulcers, rhagades, erosion, chilblains and gangrene accompanying mucocutaneous diseases or mucocutaneous disorders such as athlete' s foot, insect bites, atopic dermatitis, nummular eczema, xeroderma, seborrheic eczema, urticaria, prurigo, housewives' eczema, acne vulgaris, impetigo, folliculitis, carbuncles, furuncles, phlegmon, pyoderma, psoriasis, ichthyosis, palmoplantar keratoderma, lichen, pityriasis, wounds, burns, rhagades, erosion and chilblains; incomplete taking of skin grafts, skin flaps etc.; dental diseases such as gingivitis, alveolar pyorrhea, denture ulcers, gingival blackening and stomatitis; skin ulcers, cryesthesia and numbness due to peripheral circulatory disorders such as thromboangitis obliterans, arteriolosclerosis obliterans, diabetic peripheral circulatory disorders and lower limb varicosis; musculoskeletal diseases such as chronic rheumatoid arthritis, cervico-omo-brachial syndrome, myalgia, arthralgia and lumbago; nervous system diseases such as neuralgia, polyneuritis and subacute myelo-optic neuropathy; keratoses such as psoriasis, corns, calluses, ichthyosis, palmoplantar keratoderma, lichen and pityriasis; suppurative skin diseases such as acne vulgaris, impetigo, folliculitis, carbuncles, furuncles, phlegmon, pyoderma and suppurative eczema; suppression of regrowth of hair after depilation (treatment of unwanted hair) ; and cosmetic problems with the skin or hair such as freckles, rough skin, loss of clarity of the skin, loss of elasticity or luster of the skin, and loss of glossiness of the hair, and partial obesity. However, with such a composition, the carbon dioxide must be held in the form of bubbles, and hence to hold a sufficient amount of carbon dioxide to obtain cosmetic or medical effects, at least a certain thickness is required. Moreover, to obtain cosmetic or medical effects using the aforementioned composition, the composition must be used in large quantities for a long time period, and the effects are weak.

On the other hand, a poultice for obtaining a blood circulation promoting effect using carbon dioxide gas (i.e. gaseous carbon dioxide) is proposed in Japanese Patent Application Laid-open No. 62-286922. With this poultice, a cloth containing water is placed onto a cloth containing a carbonate and an organic acid to generate carbon dioxide gas, which is dissolved in the water contained in the cloth and is thus used as dissolved carbon dioxide gas; however, the reaction between a carbonate and an organic acid is generally very rapid, and hence the amount of carbon dioxide discharged into the atmosphere is greater than the amount dissolved in the water, and thus cosmetic or medical effects due to transdermal or transmucosal absorption of carbon dioxide cannot really be expected.

In view of this state of affairs, the objective of the present invention is to provide a material for formation of carbon dioxide external preparation in order to prepare a carbon dioxide external preparation; by which strong cosmetic and medical effects are readily obtained.

### DISCLOSURE OF THE INVENTION

A material for formation of carbon dioxide external preparation according to claim 1 is characterized by comprising a base agent that comprises a polymeric three-dimensional network structure impregnated with a viscous material containing at least an acid and water, and is made to contact with skin in use, and a reactant that contains at least a carbonate, and is made to contact with the base agent in use so as to generate carbon dioxide, the carbon dioxide dissolving in the viscous material substantially in a non-bubble state.

The polymeric three-dimensional network structure in the present invention refers to a three-dimensional network structure formed by an elastic polymer, wherein when a fluid is impregnated in then the polymer expands, and hence more of the fluid can be impregnated in without leaking out, but if a gaseous substance is generated in the fluid that has been impregnated in, then a resistance of the aforementioned polymer acts against the force of the gaseous substance trying to form bubbles, and expansion of the aforementioned polymer impregnated with the fluid is suppressed, and hence the aforementioned bubble formation of the substance is suppressed.

When the reactant containing at least a carbonate is made to contact with the base agent comprising such a polymeric three-dimensional network structure impregnated with a viscous material containing at least an acid and water, the acid in the aforementioned base agent react with the carbonate in the aforementioned reactant to generate carbon dioxide. The carbon dioxide-generating reaction between an acid and a carbonate generally proceeds rapidly in the presence of water, and hence most of the carbon dioxide generated forms bubbles and is discharged into the atmosphere and hence is not used in transdermal or transmucosal absorption, and thus cosmetic or medical effects are hard to obtain. In contrast to this, with the material for formation of carbon dioxide external preparation of the present invention, the reaction between the acid and the carbonate is suppressed by the viscosity of the viscous material in the base agent, and hence the carbon dioxide is generated gradually, and moreover due to a combined action of the viscosity, surface tension and long-flowability (a property of flowing all joined together when poured) of the viscous material, when carbon dioxide is generated in the viscous material, a resistance acting to suppress expansion of the viscous material will act, and hence the generated carbon dioxide will be suppressed from forming bubbles and being discharged into the atmosphere (hereinafter this is referred to as the 'viscous material combined action').

Furthermore, with the material for formation of carbon dioxide external preparation of the present invention, the viscous material of the aforementioned base agent is impregnated into the polymeric three-dimensional network structure, and hence when carbon dioxide is generated through contact with the aforementioned reactant, the resistance of the polymer in the aforementioned polymeric three-dimensional network structure will act against the force of the carbon dioxide trying to form bubbles, and expansion of the polymeric three-dimensional network structure will be suppressed, and hence the aforementioned bubble formation of carbon dioxide will be suppressed (hereinafter this is referred to as the 'polymer bubble formation suppressing action').

That is, with the material for formation of carbon dioxide external preparation of the present invention, carbon dioxide is generated gradually due to the viscosity of the viscous material, and moreover the viscous material combined action and the polymer bubble formation suppressing action work together so that bubble formation of the generated carbon dioxide is strongly suppressed. When carbon dioxide, which is readily soluble in water, does not form bubbles after being generated, it will immediately dissolve in the viscous material in a non-bubble state; however, the material for formation of carbon dioxide external preparation of the present invention can be made to be relatively thin and hence the volume thereof can be made low relative to the amount of carbon dioxide generated, and the excess carbon dioxide that cannot be dissolved in the viscous material, in a state that could be applied onto skin, will readily migrate into tissue such as skin and blood vessels, which contains water so that carbon dioxide readily dissolves therein, and will thus be transdermally or transmucosally absorbed with little wastage, whereby strong cosmetic and medical effects are obtained in a short time.

'Substantially in a non-bubble state' in the present invention refers to a state in which it is difficult to identify bubbles of carbon dioxide with naked eyes.

The 'contact' between the base agent and the reactant in the present invention refers to the adhesion of the base agent to the reactant such that the acid reacts with the carbonate; this includes, for example, placing the reactant on the polymeric three-dimensional network structure impregnated with the viscous material without applying an external force so that the acid and the carbonate mix and react with one another merely through gravity and osmotic force; as well as, for example, pushing the reactant against the polymeric three-dimensional network structure impregnated with the viscous material by applying an external force so that the acid and the carbonate mix and react with one another, and the like.

Unless particularly indicated, the 'skin' referred to in the present invention also includes mucous membrane.

Note that in the present invention, the acid and the carbonate could be swapped over, i.e. a material for formation of carbon dioxide external preparation comprising a base agent containing a carbonate in a viscous material and a reactant containing an acid is also possible; however, this is not very desirable, since when the base agent that directly contacts with skin were strongly alkaline then the skin might be damaged. On the other hand, healthy skin is originally weakly acidic, and hence has high resistance to acids. The material for formation of carbon dioxide external preparation of the present invention thus has the advantage of being gentle to skin.

With the aforementioned material for formation of carbon dioxide external preparation, a sheet-like structure can be used as the aforementioned polymeric three-dimensional network structure.

With the aforementioned material for formation of carbon dioxide external preparation, it is preferable for the aforementioned polymeric three-dimensional network structure to be a fibrous or porous absorbent. With the aforementioned fibrous or porous absorbent, a viscous material, whether liquid or semi-solid, can be easily impregnated therein, and moreover not only a water-soluble material, but also a water-oil dispersion such as a cream, a polymeric water-swelling material or the like can be impregnated in, and hence any of various raw materials can be used in accordance with the purpose and the usage, and thus the material for formation of carbon dioxide external preparation of the present invention can be yet more suitably used with a cosmetic or medical usage, and moreover impregnating the viscous material in is very easy. Moreover, such an absorbent can be used patched on skin, and hence handling is easy.

With the aforementioned material for formation of carbon dioxide external preparation, it is preferable for the aforementioned viscous material to contain at least one thickener selected from the group consisting of sodium alginate, propylene glycol alginate ester, carrageenan, carboxyvinyl polymers, polyvinyl alcohol and polyvinyl pyrrolidone. Such thickeners have high affinity with skin, and hence by preparing the viscous material using these, a carbon dioxide external preparation that can be used yet more suitably with a cosmetic or medical purpose can be obtained.

With the aforementioned material for formation of carbon dioxide external preparation, it is preferable for the aforementioned viscous material to be an emulsion or cream containing at least an acid, water, oil and a surfactant. In this case, the carbon dioxide external preparation obtained by making the base agent and the reactant contact one another can be used yet more suitably with a cosmetic purpose, since the moisturizing ability and the whitening effect will be strengthened.

With the aforementioned material for formation of carbon dioxide external preparation, preferably, the aforementioned viscous material contains at least one thickener selected from the group consisting of sodium alginate, propylene glycol alginate ester, carrageenan, carboxyvinyl polymers, polyvinyl alcohol and polyvinyl pyrrolidone, the acid in the aforementioned viscous material is at least one selected from the group consisting of malic acid, succinic acid, sodium dihydrogen phosphate, carboxyvinyl polymers, citric acid, L-ascorbic acid and tartaric acid, and the polymeric three-dimensional network structure is a nonwoven cloth or a sponge. With such a combination, the aforementioned viscous material combined action and the polymer bubble formation suppressing action will work together yet better, and hence particularly strong cosmetic or medical effects will be reliably obtained.

With the aforementioned material for formation of carbon dioxide external preparation, it is preferable for the reactant to be a viscous material further containing a thickener and water. When the reactant is a viscous material further containing a thickener and water, then when the reactant is made to contact with the corresponding base agent, the carbonate will migrate into the base agent gradually and hence the reaction with the acid will occur more gradually, and thus the carbon dioxide will be generated more gradually, and hence will dissolve in a non-bubble state more reliably and efficiently. Furthermore, the side of the carbon dioxide external preparation obtained facing the atmosphere will be covered by the viscous material of the reactant, and hence diffusion of the carbon dioxide into the atmosphere will be suppressed yet more strongly. As a result, in the state in which the aforementioned carbon dioxide external preparation has been applied to the skin, the carbon dioxide will be transdermally or transmucosally absorbed with yet less wastage, and hence strong cosmetic and medical effects will be obtained in a yet shorter time.

With the aforementioned material for formation of carbon dioxide external preparation, preferably, the aforementioned reactant contains water and at least one thickener selected from the group consisting of sodium alginate, propylene glycol alginate ester, carrageenan, polyvinyl alcohol and polyvinyl pyrrolidone, and the aforementioned carbonate is sodium hydrogen carbonate. With such a combination, carbon dioxide in a non-bubble state will dissolve yet more efficiently and reliably, and hence particularly strong cosmetic or medical effects will be reliably obtained.

With the aforementioned material for formation of carbon dioxide external preparation, preferably, the viscous material in the aforementioned base agent contains at least one thickener selected from the group consisting of sodium alginate, propylene glycol alginate ester, carrageenan, carboxyvinyl polymers, polyvinyl alcohol and polyvinyl pyrrolidone, the acid in the aforementioned viscous material is at least one selected from the group consisting of malic acid, succinic acid, sodium dihydrogen phosphate, carboxyvinyl polymers, citric acid, L-ascorbic acid and tartaric acid, the aforementioned polymeric three-dimensional network structure is a nonwoven cloth or a sponge, the aforementioned reactant contains water and at least one thickener selected from the group consisting of sodium alginate, propylene glycol alginate ester, carrageenan, polyvinyl alcohol and polyvinyl pyrrolidone, and the aforementioned carbonate is sodium hydrogen carbonate. With such a combination, due to a combination of the viscous material combined action and the polymer bubble formation suppressing action of the base agent, and the viscous material combined action of the viscous material of the reactant, yet stronger cosmetic or medical effects will be reliably obtained.

With the aforementioned material for formation of carbon dioxide external preparation, it is preferable for the reactant to be supported on a sheet-like or bag-shaped support. When a sheet-like or bag-shaped support having the reactant supported thereon (hereinafter referred to as a 'reactant support') is used, then preparation of the carbon dioxide external preparation is simple, and the effects can be obtained more reliably. For example, in the case that the reactant is a viscous material, the support is a sheet-like absorbent such as an nonwoven cloth, and the reactant is impregnated into the absorbent, there will be a viscous material combined action and a polymer bubble formation suppressing action, and hence strong cosmetic and medical effects will be obtained.

There are no particular limitations on the support in the present invention, so long as this support can support a solid, a semi-solid, a liquid or the like. More specifically, in the case that the reactant is, for example, a semi-solid or a liquid, a fibrous sheet-like absorbent, a porous sponge or the like in which the reactant can be impregnated can be used; in the case that the reactant is, for example, a solid such as granules and fine granules, a bag-shaped woven product having a fine mesh so as to be able to contain the solid can be used; in the case that the reactant is, for example, a sticky semi-solid such as a paste or a hydrogel, a closing covering material as described below can be used, with this covering material being taken as a backing and the reactant being spread thereover; however, there is no limitation to these examples.

With the aforementioned material for formation of carbon dioxide external preparation, it is preferable for the surface of the support on which the reactant is supported that is on the atmosphere side in use to be covered with a covering material that is impermeable to or has low permeability to carbon dioxide (hereinafter referred to as a 'closing covering material'). With the carbon dioxide external preparation obtained from a material for formation of carbon dioxide external preparation of the present invention, even as is, the carbon dioxide is strongly suppressed from forming bubbles and diffusing into the atmosphere, and hence the carbon dioxide dissolves in the viscous material substantially in a non-bubble state, and thus can be transdermally or transmucosally absorbed efficiently, whereby strong cosmetic and medical effects are obtained; however, when the aforementioned base agent is made to contact with the skin and the reactant support is placed thereon, if the surface of the reactant support on the atmosphere side is covered with the closing covering material as described above, then due to the closing effect, the carbon dioxide will be suppressed yet more strongly from forming bubbles and diffusing into the atmosphere, and hence the rate of transdermal or transmucosal absorption of carbon dioxide will be further increased, whereby stronger cosmetic and medical effects will be obtained in a yet shorter time. Note that to obtain the aforementioned closing effect, the surface of the reactant support on the atmosphere side may be covered with the closing covering material after the reactant support has been placed on the base agent, but if the surface of the reactant support that will be on the atmosphere side is covered with the closing covering material in advance, then application is easier, and the closing effect can be obtained more reliably.

By using a material for formation of carbon dioxide external preparation as described above, a carbon dioxide external preparation in which carbon dioxide is dissolved in the viscous material substantially in a non-bubble state is obtained, and according to such a carbon dioxide external preparation, strong cosmetic or medical effects due to transdermal or transmucosal absorption of carbon dioxide can be obtained.

### BRIEF DESCRIPTION OF THE DRAWINGS

FIG. 1 is a schematic sectional view of a material for formation of carbon dioxide external preparation according to an embodiment of the present invention.
FIG. 2 is an enlarged sectional view of principal parts of the base agent.
FIG. 3 is an enlarged sectional view of principal parts of the reactant.

### BEST MODE FOR CARRYING OUT THE INVENTION

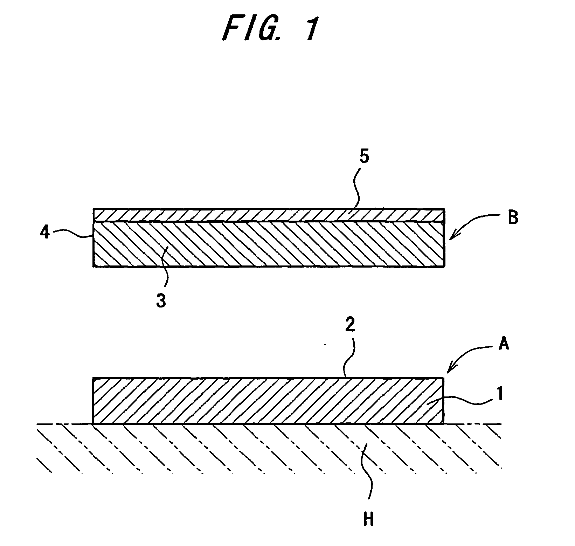

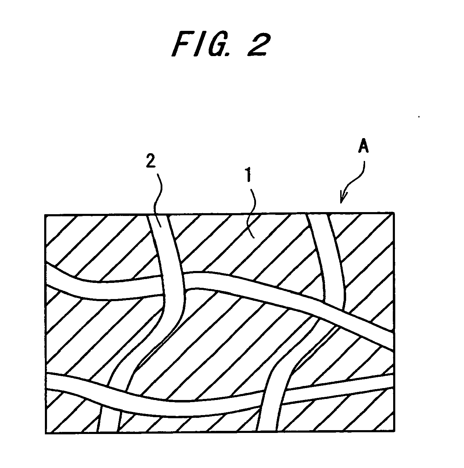

FIG. 1 is a schematic sectional view of a material for formation of carbon dioxide external preparation according to an embodiment of the present invention, and FIG. 2 is an enlarged sectional view of principal parts of the base agent A of the aforementioned material for formation of carbon dioxide external preparation. The material for formation of carbon dioxide external preparation according to the present embodiment comprises a base agent A that comprises a polymeric three-dimensional network structure 2 impregnated with a viscous material 1 containing at least an acid and water, and a reactant B that contains at least a carbonate; in use, in a state with the aforementioned base agent A patched on skin H, the reactant B is made to contact with the base agent A, whereupon carbon dioxide is generated, and this carbon dioxide dissolves in the aforementioned viscous material 1 in a non-bubble state. Note that the base agent A may, of course, be patched on a mucous membrane instead of the skin H.

The viscous material 1 of the aforementioned base agent A may be liquid or semi-solid, provided the viscosity thereof is more than 20 cps at 20°C. If the viscosity of the viscous material 1 is less than 20 cps at 20°C, then there will be a risk that when the base agent A containing the viscous material and the reactant B are made to contact one another, the viscous material combined action and the polymer bubble formation suppressing action may not be sufficiently obtained, in which case the generated carbon dioxide will form bubbles, and hence will diffuse into the atmosphere and thus will not be used sufficiently in transdermal or transmucosal absorption.

The form of the viscous material 1 can be selected as appropriate in accordance with the purpose and the usage, with examples including a viscous liquid, an ointment, a cream, a paste, and a fluid hydrogel. However, with any of these forms, a viscosity so high that migration of the carbonate in the reactant B will be difficult is undesirable. If the viscosity of the viscous material 1 is so high that migration of the carbonate in the reactant B is difficult, then when the base agent A and the reactant B come into contact with one another, carbon dioxide will not be generated sufficiently, or carbon dioxide will be generated on the surface of the aforementioned polymeric three-dimensional network structure 2, and will diffuse into the atmosphere and hence will not be used sufficiently in transdermal or transmucosal absorption; it will thus be difficult for cosmetic and medical effects to be obtained.

In the case of using a viscous liquid as the form of the viscous material 1, the viscous material 1 is suitably used as an aqueous solution, a suspension, a swelling liquid or the like obtained using at least an acid, water and a thickener. There are no particular limitations on the method of manufacturing the viscous liquid, which may be manufactured using a known method.

In the case that the viscous material 1 is made to be an ointment, the viscous material 1 can be easily manufactured merely by using an acid as one of the raw materials in a known ointment manufacturing method. To make the reaction of the acid with the carbonate 3 more reliable, it is preferable to produce a hydrophilic ointment in which the acid can dissolve in water.

In the case that the viscous material 1 is made to be a cream, the viscous material 1 can be easily manufactured merely by using an acid as one of the raw materials in a known cream manufacturing method.

A paste-like viscous material 1 can be manufactured by further adding, to the aforementioned acid and water, a thickener at a concentration such that a paste is formed.

A hydrogel-like viscous material 1 can be manufactured by further adding a hydrogel-forming raw material to the aforementioned acid and water. There are no particular limitations on the hydrogel-forming raw material; examples thereof include natural polymers such as alginic acid, amylose, casein, carrageenan, xanthan gum, karaya gum, dextran, chitin, starch, fibrin, pullulan, gelatin and mannan; semi-synthetic polymers such as sodium alginate, propylene glycol alginate ester, methyl cellulose, ethyl cellulose, hydroxymethyl cellulose, starch-acrylic acid copolymers and hydroxypropyl cellulose; and synthetic polymers such as carboxyvinyl polymers, polyvinyl alcohol, polyvinyl pyrrolidone, polyacrylic acid, sodium polymethacrylate, polyacrylamide and isopropylacrylamide-butyl methacrylate copolymers; and one or more of these can be used.

In the case of sodium alginate, when the acid is a strong acid, a fluid hydrogel is formed with this alone; in the case of using a weak acid, a calcium alginate hydrogel can easily be formed by using a water-soluble calcium salt. A calcium alginate hydrogel has a relatively high hardness, and hence impregnating into the polymeric network structure 2 as it stands is difficult; it is thus preferable to first impregnate a sodium alginate sol therein, and then apply on an aqueous solution of a water-soluble calcium salt, or else immerse the polymeric network structure 2 that has been impregnated with the sodium alginate sol in an aqueous solution of a water-soluble calcium salt.

The aforementioned hydrogel-like viscous material 1 will itself form a loose three-dimensional network structure, and hence even in the case that the gel hardness of the hydrogel is not so high, when this hydrogel is further impregnated into the polymeric three-dimensional network structure 2, a polymer bubble formation suppressing action of the polymeric three-dimensional network structure 2 will act together with a viscous material combined action and a polymer bubble formation suppressing action of the hydrogel in additive or synergistic fashion, and hence the rate of generation of carbon dioxide in the hydrogel will be suitably suppressed, and thus the carbon dioxide will be more strongly suppressed from forming bubbles and diffusing into the atmosphere.

As a thickener used in the viscous material 1, at least one selected from the group consisting of natural polymers, semi-synthetic polymers, synthetic polymers and inorganic materials can be used.

Examples of natural polymers include plant-based polymers such as gum arabic, carrageenan, galactan, agar, xanthan gum, quince seed gum, guar gum, tragacanth, pectin, mannan, locust bean gum, wheat starch, rice starch, corn starch and potato starch; microorganism-based polymers such as curdlan, xanthan gum, succinoglucan, dextran, hyaluronic acid and pullulan; and protein-type polymers such as albumin, casein, collagen, gelatin and fibroin; and one or more of these can be used. Of these, from the standpoint of affinity with the skin and so on, gum arabic, carrageenan, xanthan gum, tragacanth, hyaluronic acid, pullulan, pectin, mannan and locust bean gum can be preferably used, and from the standpoint of feeling in use and so on, carrageenan can be more preferably used.

Examples of semi-synthetic polymers include cellulose type polymers such as ethyl cellulose, carboxymethyl cellulose, carboxymethyl ethyl cellulose, carboxymethyl starch, croscarmellose, crystalline cellulose, cellulose acetate, cellulose acetate phthalate, hydroxyethyl cellulose, hydroxypropyl cellulose, hydroxypropyl methyl cellulose, hydroxypropyl methyl cellulose phthalate, methyl cellulose and methyl hydroxypropyl cellulose; starch-type polymers such as pregelatinized starch, partially pregelatinized starch, carboxymethyl starch, dextrin, methyl starch, starch-acrylic acid copolymers and cellulose-acrylonitrile graft copolymers; alginate-type polymers such as sodium alginate and propylene glycol alginate ester; and other polysaccharide-type polymers such as chondroitin sulfate sodium and sodium hyaluronate; and one or more of these can be used. Of these, from the standpoint of affinity with the skin and so on, sodium alginate, propylene glycol alginate ester, sodium carboxymethyl cellulose, dextrin and sodium hyaluronate can be preferably used, and from the standpoint of feeling in use and so on, sodium alginate and propylene glycol alginate ester can be more preferably used.

Examples of synthetic polymers include carboxyvinyl polymers, sodium polyacrylate, polyamines, polyacrylamide, polyvinylacetal diethylaminoacetate, polyvinyl alcohol, polyvinyl pyrrolidone, polyethylene glycol, polyethylene oxide, poly(meth)acrylic acid, polyvinyl methyl ether and so on; and one or more of these can be used. Of these, from the standpoint of affinity with the skin and soon, carboxyvinylpolymers, sodium polyacrylate, polyacrylamide, polyvinyl alcohol and polyvinyl pyrrolidone can be preferably used, and from the standpoint of feeling in use and so on, carboxyvinyl polymers, polyvinyl alcohol and polyvinyl pyrrolidone can be more preferably used.

Examples of inorganic materials include hydrated silica, silica, colloidal alumina, bentonite, laponite and so on; and one or more of these can be used.

The blending amount of acid in the aforementioned viscous material 1 is preferably in a range of 0.05 to 30 wt%, more preferably 0.5 to 10 wt%, relative to the total amount of the viscous material 1. When this amount is less than 0.05 wt%, then however high the amount of the carbonate 3 that reacts with the acid is, the amount of carbon dioxide generated will be low, and hence cosmetic or medical effects will be hard to obtain. When the amount exceeds 30 wt%, then even if the amount of the carbonate 3 that reacts with the acid is high and a large amount of carbon dioxide is generated, increased effects will be hard to obtain.

There are no particular limitations on the acid used in the viscous material 1, with it being possible to use one or more selected from the group consisting of inorganic acids and organic acids. Examples of inorganic acids include hydrochloric acid, nitric acid, sulfuric acid, phosphoric acid, potassium dihydrogen phosphate, sodium dihydrogen phosphate, sodium sulfite, potassium sulfite, sodium pyrosulfite, potassium pyrosulfite, acidic sodium hexametaphosphate, acidic potassium hexametaphosphate, acidic sodium pyrophosphate, acidic potassium pyrophosphate, sulfamic acid and so on; and one or more of these can be used.

Examples of organic acids include straight chain fatty acids such as formic acid, acetic acid, propionic acid, butyric acid and valeric acid; dicarboxylic acids such as oxalic acid, malonic acid, succinic acid, glutaric acid, adipic acid, pimelic acid, fumaric acid, maleic acid, phthalic acid, isophthalic acid and terephthalic acid; acidic amino acids such as glutamic acid and aspartic acid; and oxyacids such as glycolic acid, malic acid, tartaric acid, citric acid, lactic acid, hydroxyacrylic acid, α-oxybutyric acid, glyceric acid, tartronic acid, salicylic acid, gallic acid, tropic acid, ascorbic acid and gluconic acid; and one or more of these can be used.

The blending amount of water in the aforementioned viscous material 1 is preferably as high as possible, so long as the viscosity of the viscous material 1 is not less than 20 cps at 20°C. When the blending amount of water is low, then there may be insufficient migration of the carbonate into the viscous material 1, and hence the required amount of carbon dioxide may not be generated.

The viscous material 1 can be impregnated into the polymeric three-dimensional network structure 2 by known means. The amount of the viscous material 1 impregnated into the polymeric three-dimensional network structure 2 will depend on the thickness of the polymeric three-dimensional network structure 2, but is preferably not less than approximately 0.01 g per square centimeter. However, when the amount impregnated in is too high such that the polymeric three-dimensional network structure 2 is completely swamped, then it will be difficult to obtain the polymer bubble formation suppressing action, and hence it will be difficult to obtain cosmetic or medical effects. When the amount impregnated in is less than 0.01 g per square centimeter, then upon contact with the reactant B, the amount of carbon dioxide generated will be low, and hence it will be difficult to obtain cosmetic or medical effects.

There are no particular limitations on the aforementioned polymeric three-dimensional network structure 2, so long as a fluid material such as a viscous material can be impregnated therein, or an aqueous solution can be impregnated therein to form a viscous material, and application to the skin is possible. In the embodiment shown in the drawings, a sheet-like fibrous or porous absorbent is used as the aforementioned polymeric three-dimensional network structure 2.

The aforementioned absorbent may be any of a woven cloth, a nonwoven cloth, a sponge or the like, and can be selected as appropriate in accordance with the specific purpose, site of usage and so on; however, a nonwoven cloth is light, and has excellent shrinkage and crease resistance, and is thus particularly preferable.

Any of natural fibers, synthetic fibers or semi-synthetic fibers can be used as a material of a fibrous absorbent. Examples of natural fibers include plant fibers such as cotton and hemp; and animal fibers such as wool, silk and natural sponge, examples of synthetic fibers include nylon, vinylon, Tetron, acrylics and polyester, and examples of semi-synthetic fibers include recycled fibers such as viscose rayon, and semi-synthetic fibers such as acetate rayon; and one or more of these can be used. Of these, fibers made of a polymer that has an elastic three-dimensional network structure and has many hydrophilic groups in the molecule thereof and is thus commonly known as a water-absorbing polymer can be suitably used.

The thickness of the polymeric three-dimensional network structure 2 is preferably in a range of 0.1 to 10 mm, more preferably 0.1 to 5 mm, yet more preferably 0.1 to 2 mm. When the thickness is less than 0.1 mm, then the amount of the viscous material 1 contained per unit area is low, and hence even if all of the acid contained therein reacts with the carbonate 3, the amount of carbon dioxide generated will be low, and hence sufficient cosmetic or medical effects may not be obtained. When the thickness is too high, then even if all of the acid contained therein reacts with the carbonate 3 to generate a large amount of carbon dioxide, increased cosmetic or medical effects will not be obtained, and moreover there may be a lack of convenience as an external preparation.

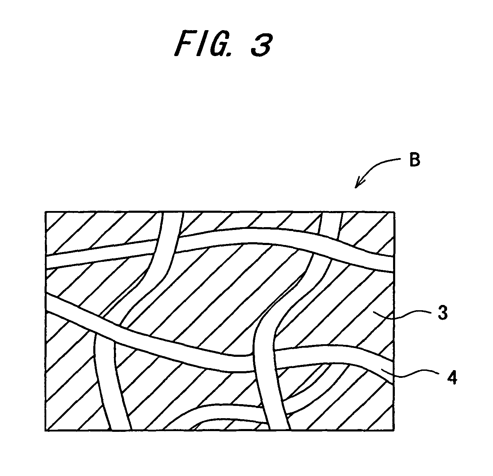

Referring to FIG. 3, there are no particular limitations on the aforementioned reactant B, so long as the reactant B is such that the carbonate 3 will migrate into the viscous material 1 contained in the base agent A when the reactant B is made to contact with the base agent A; the reactant B is used in the form of a solid, a semi-solid, a liquid or the like in accordance with the purpose and the usage. There are no particular limitations on the form. In the case of a solid, granules, fine granules, a powder or the like can be used, in the case of a semi-solid, an ointment, a cream, a paste, a hydrogel or the like can be used, and in the case of a liquid, an ordinary aqueous solution, a viscous liquid or the like can be used. However, in the case of a hydrogel, when the hardness is too high, then upon contact with the base agent A, it may take too much time for the carbonate contained in the reactant B to migrate into the viscous material of the base agent A, and hence generation of carbon dioxide may be too slow, and thus sufficient cosmetic or medical effects may not be obtained; a fluid hydrogel is thus preferable.

In the example shown in the drawings, the reactant B is supported on a sheet-like support 4. There are no particular limitations on the support 4; in the case that the reactant B is liquid or semi-solid, the polymeric three-dimensional network structure as described earlier can be used, and in the case that the reactant B is a viscous semi-solid, a closing covering material can be used. Moreover, in the case that the reactant B is solid, a bag-shaped support 4 comprising a woven product having a mesh finer than the particle size of the solid can be used.

There are no particular limitations on a polymeric three-dimensional network structure used in the aforementioned support 4, with it being possible to use the same form and material as with the polymeric three-dimensional network structure 2 used in the base agent A.

Moreover, there are no particular limitations on the aforementioned closing covering material, with it being possible to use anything made of a material that completely stops the permeation of carbon dioxide therethrough, or through which carbon dioxide permeates only with difficulty. Examples thereof include nylon, vinylon, a composite between nylon and ethylene-vinyl alcohol, polyethylene, polyethylene naphthalate, polyvinylidene chloride, a polyamide between metaxylylene diamine and adipic acid, polyacrylonitrile, ethylene-vinyl alcohol, and polypropylene; and one or more of these can be used.

Furthermore, there are no particular limitations on the aforementioned bag-shaped woven product, so long as the mesh is finer than the particle size of the reactant B contained therein; there are no particular limitations on the material, with it being possible to use any of natural fibers, synthetic fibers or semi-synthetic fibers as used for the material of the absorbent described earlier.

There are no particular limitations on the carbonate 3 used in the reactant B, so long as carbon dioxide is generated upon reaction with an acid. Examples thereof include ammonium carbonate, ammonium hydrogen carbonate, potassium carbonate, potassium hydrogen carbonate, potassium sesquicarbonate, sodium carbonate, sodium hydrogen carbonate, sodium sesquicarbonate, lithium carbonate, lithium hydrogen carbonate, lithium sesquicarbonate, cesium carbonate, cesium hydrogen carbonate, cesium sesquicarbonate, magnesium carbonate, magnesium hydrogen carbonate, calcium hydrogen carbonate, calcium carbonate, magnesium carbonate hydroxide, and barium carbonate; and one or more of these can be used. The blending amount of the carbonate 3 is preferably 0.05 to 30 wt%, more preferably 0.5 to 10 wt%, relative to the total amount of the reactant B. When the blending amount of the carbonate 3 is less than 0.05 wt%, then however high the amount of the acid that reacts with the carbonate 3 is, the amount of carbon dioxide generated is low, and hence cosmetic or medical effects may not be obtained. When the amount exceeds 30 wt%, then even if the amount of the acid that reacts with the carbonate 3 is high and a large amount of carbon dioxide is generated, increased effects may not be obtained.

With the aforementioned reactant B, the surface of the support 4 that will be on the atmosphere side in use (the upper surface in FIG. 1) is covered with a closing covering material 5, which is a covering material that is impermeable to or has low permeability to carbon dioxide. A sheet-like or film-like material can be used as the closing coveringmaterial 5. As a result, diffusion of carbon dioxide from the reactant B is suppressed, and hence the utilization rate of the carbon dioxide in transdermal or transmucosal absorption is increased, whereby the cosmetic and medical effects become stronger.

For covering the support 4 with the aforementioned closing covering material 5, known covering methods such as fusion using heat, microwaves, ultrasound or the like, and bonding using an adhesive, and the like can be used with no particular limitations thereon in accordance with the materials of the closing covering material 5 and the support 4 and so on.

The amounts of the aforementioned base agent A and the reactant 3 are preferably such that the amount of the carbonate 3 contained in the reactant B is in a range of 0.5 to 10 parts by weight, more preferably 1 to 5 parts by weight, per 1 part by weight of the acid contained in the base agent A.

As an example of a method of using the carbon dioxide external preparation, a method in which the base agent A and the reactant B are made to contact one another in advance to prepare the carbon dioxide external preparation and then the preparation is applied to the skin can be mentioned. As a more preferable method of use, a method in which the base agent A is patched on the skin H and then the reactant B is placed thereon can be mentioned. By this method, the carbon dioxide is used in transdermal or transmucosal absorption with little wastage immediately after having been generated.

The time for which the carbon dioxide external preparation is used will vary according to the purpose, the responsiveness of the individual to carbon dioxide and so on, but more than 1 minute is preferable, more than 5 minutes being more preferable. At less than 1 minute, sufficient cosmetic or medical effects will not be obtained. There is no problem in using the carbon dioxide external preparation for a long time, but in general once the generation of carbon dioxide has ceased, no particular effects will be obtained by using for longer than this.

The reactant B can be formed alone without being supported on a support 4; the form in this case can be selected as appropriate in accordance with the purpose and the usage, with examples thereof including granules, fine granules, a powder and tablets, and manufacture thereof can be carried out following a known manufacturing method, with there being no particular limitations. In the case that the reactant B is made to be a solid, to keep down the rate of migration of the carbonate 3 into the viscous material 1 and thus slow down the generation of carbon dioxide, it is preferable to make the reactant B contain at least the carbonate 3 and a dispersant (hereinafter referred to as a 'carbonate/dispersant-containing reactant'), with the carbonate 3 and the dispersant being mixed together as uniformly as possible.

There are no particular limitations on the dispersant, with it being possible to use sucrose, lactose, glucose, mannitol, calcium phosphate, dextrin, urea or the like as used as a dispersant in ordinary medicines and so on, and a water-soluble dispersant such as sucrose, lactose, glucose, mannitol or urea is preferable. The blending amount of the dispersant is preferably 5 to 90 wt%, more preferably 10 to 70 wt%, relative to the total amount of the carbonate/dispersant-containing reactant B. When the blending amount of the dispersant is less than 5 wt%, then it may not be possible to sufficiently keep down the rate of migration of the carbonate 3 into the viscous material 1. When the blending amount of the dispersant exceeds 90 wt%, then to generate the required amount of carbon dioxide, the carbonate/dispersant-containing reactant B have to be used in a large amount, which is inconvenient, and moreover depending on the type of the dispersant, the amount of the aforementioned reactant B may be too great that the required amount of the carbonate does not migrate into the viscous material 1, and hence a sufficient amount of carbon dioxide is not generated, and thus cosmetic or medical effects cannot be sufficiently obtained. Note that when a dispersant is not used, and the reactant B comprising only the carbonate 3 and not containing any other additives whatsoever is made to contact with the base agent A, then carbon dioxide may rapidly formbubbles on the surface of the aforementioned polymeric three-dimensional network structure 2 and diffuse into the atmosphere, so that cosmetic or medical effects are not sufficiently obtained.

A thickener may be used as the dispersant in the aforementioned carbonate/dispersant-containing reactant B. When such a carbonate/dispersant-containing reactant B in which the thickener is used as the dispersant is made to contact with the viscous material 1 of the base agent A, in addition to the above effect of the dispersant slowing down the rate of migration of the carbonate 3 into the viscous material 1, an effect of the thickener forming a film that is impermeable to or has low permeability to gas and thus effect of suppressing diffusion of carbon dioxide into the atmosphere will also be obtained, and hence the carbon dioxide will be transdermally or transmucosally absorbed with little wastage, whereby stronger cosmetic and medical effects will be obtained. There are no particular limitations on the thickener, with it being possible to use a thickener as used in the viscous material 1 described earlier. Of course, if only part of the dispersant used in ordinary medicines and so on such as sucrose, lactose, glucose, mannitol, calcium phosphate or dextrin is replaced with the aforementioned thickener, then again similar effects will be obtained.

In the case that the reactant B is made to be a semi-solid, this can be manufactured merely by using the carbonate 3 as one of the raw materials of an ordinary semi-solid such as an ointment or cream, with there being no particular limitations.

In the case that the reactant B is made to be a liquid, the liquid maybe made to be merely an aqueous solution. It is preferable to make the liquid be a viscous liquid, since then the reaction of the acid with the carbonate 3 upon contact with the base agent A will be further suppressed, and hence carbon dioxide will be generated more gradually, and moreover the surface tension will be high, and hence bubble formation and diffusion of the generated carbon dioxide will be further suppressed, and thus dissolving of the carbon dioxide in a non-bubble state will be further promoted. As a viscous liquid, a viscous liquid comprising an aqueous solution, a suspension, a swelling liquid or the like obtained using at least the carbonate 3, a thickener and water (hereinafter referred to as a 'carbonate/thickener-containing viscous reactant B') can be suitably used.

Moreover, the carbonate/thickener-containing viscous reactant B may be made into a paste or a hydrogel by increasing the blending amount of the thickener. In the case of a hydrogel, depending on the type of the thickener, a hardened gel may be produced by a method in which the reactant B is heated and then cooled, a method in which the reactant B is repeatedly frozen and thawed, a method in which the thickener in the reactant B is crosslinked using a suitable chemical crosslinking agent, a method in which the thickener is cross linked by irradiating the reactant B with ionizing radiation, or the like. Such a hardened gel can be suitably used in the form of a sheet or the like as is without being supported on a support 4. The blending amount of the thickener can be selected as appropriate in accordance with the properties of the thickener used and so on such that the viscous liquid, paste, hydrogel or hardened gel is formed. As the thickener, out of thickeners that can be used in the viscous material of the base agent, one that will not react with the carbonate in the reactant to generate carbon dioxide can be suitably used.

With the carbon dioxide external preparation, in addition to the essential components described above, raw materials commonly used in ordinary external preparations, cosmetics and so on, for example, fragrances, colorants, preservatives, surfactants, oils, moisturizers, alcohols, antioxidants, sequestering agents, anti-colorants, ultraviolet absorbing/scattering agents, vitamins, amino acids, melanin pigment synthesis inhibiting agents, nutrients, anti-inflammatories, vasodilators, hormones, astringents, antihistamines, sebum inhibiting agents, keratin stripping/dissolving agents, anti-seborrheic agents, anti-itching agent, and so on, can be blended into the viscous material or the reactant as required so long as this is within a range such that the effects of the present invention are not impaired, whereby the carbon dioxide external preparation can be used yet more suitably as a cosmetic or medicinal external preparation.

With the carbon dioxide external preparation, carbon dioxide is generated through reaction of an acid with a carbonate 3, and hence opposite to the embodiment described above, it is possible to constitute the base agent from a polymeric three-dimensional network structure impregnated with a viscous material containing at least a carbonate and water, and constitute the reactant so as to contain at least an acid. However, skin is generally easily damaged by alkalis, and hence thorough care will be required in use.

Following is a more concrete description of the present invention, giving examples; however, the present invention is not limited to these examples.

### Example 1

### (Preparation of base agent)

A fluid hydrogel-like viscous material was prepared using 1 part by weight of malic acid as an acid, 3 parts by weight of sodium alginate as a thickener, and 96 parts by weight of purified water as water, and using a 0.4 mm-thick polyester nonwoven cloth as a polymeric three-dimensional network structure, 0.03 g of the aforementioned viscous material per square centimeter of the nonwoven cloth was impregnated therein, thus preparing a base agent.

### (Preparation of reactant)

A liquid reactant was prepared using 1 part by weight of sodium hydrogen carbonate as a carbonate, and 99 parts by weight of purified water as water.

The base agent and the reactant obtained were combined to produce a material for formation of carbon dioxide external preparation.

### Example 2

### (Preparation of base agent)

A fluid hydrogel-like viscous material was prepared using 1 part by weight of succinic acid as an acid, 3 parts by weight of sodium alginate as a thickener, 0.1 part by weight of methylparaben as a preservative, and 95.9 parts by weight of purified water as water, and using a 0.4 mm-thick polyester nonwoven cloth as a polymeric three-dimensional network structure, 0.03 g of the aforementioned viscous material per square centimeter of the nonwoven cloth was impregnated therein, thus preparing a base agent.

### (Preparation of reactant)

A viscous liquid reactant was prepared using 1 part by weight of sodium sesquicarbonate as a carbonate, 3 parts by weight of sodium alginate as a thickener, 0.1 part by weight of methylparaben as a preservative, and 95. 9 parts by weight of purified water as water.

The base agent and the reactant obtained were combined to produce a material for formation ofcarbon dioxide external preparation.

### Example 3

### (Preparation of base agent)

A viscous material was prepared using 0.5 part by weight of citric acid as an acid, 3 parts by weight of propylene glycol alginate ester as a thickener, 0.1 part by weight of methylparaben as a preservative, and 96.4 parts by weight of purified water as water, and using a 0.4 mm-thick polyester nonwoven cloth as a polymeric three-dimensional network structure, 0.03 g of the aforementioned viscous material per square centimeter of the nonwoven cloth was impregnated therein, thus preparing a base agent.

### (Preparation of reactant support)

A viscous material was prepared using 0.5 part by weight of sodium carbonate as a carbonate, 2 parts by weight of carrageenan as a thickener, 0.1 part by weight of methylparaben as a preservative, and 97.4 parts by weight of purified water as water, and using a 0.4 mm-thick polyester nonwoven cloth as a support, 0.03 g of the aforementioned viscous material per square centimeter of the nonwoven cloth was supported thereon, thus preparing a reactant support supporting a reactant.

The base agent and the reactant support obtained were combined to produce a material for formation of carbon dioxide external preparation.

### Example 4

### (Preparation of base agent)

A fluid hydrogel-like viscous material was prepared using 1 part by weight of malic acid as an acid, 3 parts by weight of sodium alginate as a thickener, 0.1 part by weight of methylparaben as a preservative, and 95.9 parts by weight of purified water as water, and using a 0.4 mm-thick polyester nonwoven cloth as a polymeric three-dimensional network structure, 0.03 g of the aforementioned viscous material per square centimeter of the nonwoven cloth was impregnated therein, thus preparing a base agent.

### (Preparation of reactant support)

Using the reactant in Example 2, and a 0.4 mm-thick polyester nonwoven cloth having a polyethylene film laminated thereon as a closing covering material, 0.03 g of the aforementioned reactant per square centimeter of the nonwoven cloth was supported thereon, thus preparing a reactant support supporting a reactant.

The base agent and the reactant support obtained were combined to produce a material for formation of carbon dioxide external preparation.

### Example 5

### (Preparation of base agent)

A viscous material was prepared using 1 part by weight of L-ascorbic acid as an acid, 3 parts by weight of polyvinyl alcohol as a thickener, 0.1 part by weight of methylparaben as a preservative, and 95.9 parts by weight of purified water as water, and using a 1 mm-thick polyurethane sponge as a polymeric three-dimensional network structure, 0.03 g of the aforementioned viscous material per square centimeter of the sponge was impregnated therein, thus preparing a base agent.

### (Preparation of reactant support)

Using the reactant in Example 2, and a 0.4 mm-thick polyester nonwoven cloth having a polyethylene film laminated thereon as a closing covering material, 0.03 g of the aforementioned reactant per square centimeter of the nonwoven cloth was supported thereon, thus preparing a reactant support supporting a reactant.

The base agent and the reactant support obtained were combined to produce a material for formation of carbon dioxide external preparation.

### Example 6

### (Preparation of base agent)

A viscous material was prepared using 1 part by weight of tartaric acid as an acid, 3 parts by weight of polyvinyl pyrrolidone as a thickener, 0.1 part by weight of methylparaben as a preservative, and 95.9 parts by weight of purified water as water, and using a 0.4 mm-thick polyester nonwoven cloth as a polymeric three-dimensional network structure, 0.03 g of the aforementioned viscous material per square centimeter of the nonwoven cloth was impregnated therein, thus preparing a base agent.

### (Preparation of reactant)

A viscous material was prepared using 2 parts by weight of sodium hydrogen carbonate as a carbonate, 6 parts by weight of sodium alginate as a thickener, 0.1 part by weight of methylparaben as a preservative, and 91.9 parts by weight of purified water at 60°C as water, and then the viscous material was cooled to 2°C, thus preparing a hydrogel-like reactant.

The base agent and the reactant obtained were combined to produce a material for formation of carbon dioxide external preparation.

### Example 7

### (Preparation of base agent)

An aqueous solution was prepared using 1 part by weight of malic acid as an acid, 5 parts by weight of sodium alginate as a thickener, 0.1 part by weight of methylparaben as a preservative, and 93.9 parts by weight of purified water at 60°C as water. This aqueous solution was cooled to 2°C, thus preparing a fluid hydrogel-like viscous material, and using a 0.4 mm-thick polyurethane sponge as an absorbent, 0.06 g of the aforementioned viscous material per square centimeter of the sponge was impregnated therein, thus preparing a base agent.

### (Preparation of reactant support)

A viscous material was prepared using 1 part by weight of sodium hydrogen carbonate acid as a carbonate, 3 parts by weight of carrageenan as a thickener, 0.1 part by weight of methylparaben as a preservative, and 95.9 parts by weight of purified water as water, and using a polyethylene film as a closing covering material, 0. 03 g of the aforementioned viscous material per square centimeter of the closing covering material was applied and thus supported thereon, thus preparing a reactant support.

The base agent and the reactant support obtained were combined to produce a material for formation of carbon dioxide external preparation.

### Example 8

### (Preparation of base agent)

A cream-like viscous material was prepared following an ordinary method using 1 part by weight of malic acid as an acid, 53.9 parts by weight of purified water as water, 6 parts by weight of stearyl alcohol, 2 parts by weight of stearic acid, 3 parts by weight of hydrogenated lanolin, 9 parts by weight of squalane and 10 parts by weight of octyl dodecanol as oils, 6 parts by weight of 1,3-butylene glycol and 4 parts by weight of PEG 1500 as moisturizers, 2 parts by weight of glyceryl monostearate and 3 parts by weight of POE (25) cetyl alcohol ether as surfactants, and 0.1 part by weight of methylparaben as a preservative, and using a 0.4 mm-thick polyester nonwoven cloth as a polymeric three-dimensional network structure, 0.03 g of the aforementioned viscous material per square centimeter of the nonwoven cloth was impregnated therein , thus preparing a base agent.

### (Preparation of reactant)

A cream-like reactant was prepared following an ordinary method using 1 part by weight of sodium hydrogen carbonate as a carbonate, 53. 9 parts by weight of purified water as water, 6 parts by weight of stearyl alcohol, 2 parts by weight of stearic acid, 3 parts by weight of hydrogenated lanolin, 9 parts by weight of squalane and 10 parts by weight of octyl dodecanol as oils, 6 parts by weight of 1,3-butylene glycol and 4 parts by weight of PEG 1500 as moisturizers, 2 parts by weight of glyceryl monostearate and 3 parts by weight of POE (25) cetyl alcohol ether as surfactants, and 0.1 part by weight of methylparaben as a preservative.

The base agent and the reactant obtained were combined to produce a material for formation of carbon dioxide external preparation.

### Example 9

### (Preparation of base agent)

Using the viscous material in Example 1, and a 0.4 mm-thick cotton facemask type nonwoven cloth as a polymeric three-dimensional network structure, 0.03 g of the aforementioned viscous material per square centimeter of the nonwoven cloth was impregnated therein, thus preparing a base agent.

### (Preparation of reactant support)

Using the reactant in Example 1, and as a support a 0. 4 mm-thick cotton facemask type nonwoven cloth having a polyethylene film thereon as a backing, 0.03 g of the aforementioned reactant per square centimeter of the nonwoven cloth was supported thereon, thus preparing a reactant support supporting a reactant.

The base agent and the reactant support obtained were combined to produce a material for formation of carbon dioxide external preparation.

### Example 10

### (Preparation of base agent)

A fluid hydrogel-like viscous material was prepared using 1 part by weight of malic acid as an acid, 3 parts by weight of sodium alginate as a thickener, and 96 parts by weight of purified water as water, and using a 1 mm-thick polyester/cotton mixture nonwoven cloth as a polymeric three-dimensional network structure, 0. 03 g of the aforementioned viscous material per square centimeter of the nonwoven cloth was impregnated therein, thus preparing a base agent.

### (Preparation of reactant)

A viscous liquid reactant was prepared using 1 part by weight of sodium hydrogen carbonate as a carbonate, 1 part by weight of sodium alginate as a thickener, and 98 parts by weight of purified water as water.

The base agent and the reactant obtained were combined to produce a material for formation ofcarbon dioxide external preparation.

### Example 11

### (Preparation of base agent)

A viscous material was prepared using 1 part by weight of succinic acid as an acid, 3 parts by weight of propylene glycol alginate ester as a thickener, 0.1 part by weight of methylparaben as a preservative, and 95.9 parts by weight of purified water as water, and using a 0.4 mm-thick polyester nonwoven cloth as a polymeric three-dimensional network structure, 0.03 g of the aforementioned viscous material per square centimeter of the nonwoven cloth was impregnated therein, thus preparing a base agent.

### (Preparation of reactant)

A viscous liquid reactant was prepared using 1 part by weight of potassium hydrogen carbonate as a carbonate, 3 parts by weight of sodium alginate as a thickener, 0.1 part by weight of methylparaben as a preservative, and 95.9 parts by weight of purified water as water.

The base agent and the reactant obtained were combined to produce a material for formation of carbon dioxide external preparation.

### Example 12

### (Preparation of base agent)

A viscous material was prepared using 1 part by weight of sodium dihydrogen phosphate as an acid, 3 parts by weight of propylene glycol alginate ester as a thickener, 0.1 part by weight of methylparaben as a preservative, and 95.9 parts by weight of purified water as water, and using a 0.4 mm-thick cotton nonwoven cloth as a polymeric three-dimensional network structure, 0.03 g of the aforementioned viscous material per square centimeter of the nonwoven cloth was impregnated therein, thus preparing a base agent.

### (Preparation of reactant support)

A viscous material was prepared using 0.5 part by weight of sodium carbonate as a carbonate, 2 parts by weight of carrageenan as a thickener, 0.1 part by weight of methylparaben as a preservative, and 97.4 parts by weight of purified water as water, and using a 0.4 mm-thick cotton nonwoven cloth as a support, 0.03 g of the aforementioned viscous material per square centimeter of the nonwoven cloth was supported thereon, thus preparing a reactant support.

The base agent and the reactant support obtained were combined to produce a material for formation of carbon dioxide external preparation.

### Example 13

### (Preparation of base agent)

A viscous material was prepared using 1 part by weight of a carboxyvinyl polymer as an acid and a thickener, 2 parts by weight of sodium alginate as a thickener, 0.1 part by weight of methylparaben as a preservative, and 96.9 parts by weight of purified water as water, and using a 0.4 mm-thick polyester nonwoven cloth as a polymeric three-dimensional network structure, 0.03 g of the aforementioned viscous material per square centimeter of the nonwoven cloth was impregnated therein, thus preparing a base agent.

### (Preparation of reactant support)

Using the reactant in Example 10, and a 0. 4 mm-thick polyester nonwoven cloth having a polyethylene film laminated thereon as a closing covering material, 0.03 g of the aforementioned reactant per square centimeter of the nonwoven cloth was supported thereon, thus preparing a reactant support.

The base agent and the reactant support obtained were combined to produce a material for formation of carbon dioxide external preparation.

### Example 14

### (Preparation of base agent)

A viscous liquid was prepared using 1 part by weight of citric acid as an acid, 5 parts by weight of polyvinyl alcohol and 10 parts by weight of polyvinyl pyrrolidone as thickeners, 0.1 part by weight of methylparaben as a preservative, and 84.9 parts by weight of purified water as water, and using a 0.4 mm-thick cotton nonwoven cloth as a polymeric three-dimensional network structure, 0.03 g of the aforementioned viscous material per square centimeter of the nonwoven cloth was impregnated therein, thus preparing a base agent.

### (Preparation of reactant)

Using 5 parts by weight of sodium hydrogen carbonate as a carbonate, 90 parts by weight of mannitol as a dispersant, and 5 parts by weight of potato starch as a binder, a fine granular reactant was prepared using a wet granulation method with water as a solvent.

The base agent and the reactant obtained were combined to produce a material for formation of carbon dioxide external preparation.

### Example 15

### (Preparation of base agent)

Using the fluid hydrogel-like viscous material in Example 10, and a 1 mm-thick polyurethane sponge as a polymeric three-dimensional network structure, 0.06 g of the aforementioned viscous material per square centimeter of the sponge was impregnated therein, thus preparing a base agent.

### (Preparation of reactant)

A cream-like reactant was prepared using 1 part by weight of sodium hydrogen carbonate as a carbonate, 53.9 parts by weight of purified water as water, 4 parts by weight of stearic acid, 12 parts by weight of cetyl alcohol, 2 parts by weight of glyceryl monostearate and 3 parts by weight of POE (20) hardened castor oil as surfactants, 10 parts by weight of squalane and 4 parts by weight of jojoba oil as oils, 6 parts by weight of 1,3-butylene glycol and 4 parts by weight of polyethylene glycol as other raw materials, and 0.1 part by weight of methylparaben as a preservative.

The base agent and the reactant obtained were combined to produce a material for formation of carbon dioxide external preparation.

### Example 16

### (Preparation of base agent)

An emulsion-like viscous material was prepared using 1 part by weight of citric acid as an acid, 1 part by weight of cetyl alcohol, 2.5 parts by weight of white vaseline and 6 parts by weight of squalane as oils, 2 parts by weight of stearic acid, 1 part by weight of POE (20) hardened castor oil and 1 part by weight of glyceryl monostearate as surfactants, 35.9 parts by weight of distilled water as water, 0.1 part by weight of methylparaben as a preservative, and 4 parts by weight of glycerol, 0.5 part by weight of sodium carboxymethyl cellulose and 5 parts by weight of ethanol as other raw materials, and using a 0.4 mm-thick cotton nonwoven cloth as a polymeric three-dimensional network structure, 0.03 g of the aforementioned viscous material per square centimeter of the nonwoven cloth was impregnated therein, thus preparing a base agent.

### (Preparation of reactant support)

A viscous material was prepared using 2 parts by weight of sodium hydrogen carbonate as a carbonate, 4 parts by weight of sodium alginate as a thickener, 0.1 part by weight of methylparaben as a preservative, and 93. 9 parts by weight of purified water as water, and using a 0.4 mm-thick cotton nonwoven cloth as a support, 0.03 g of the aforementioned viscous material per square centimeter of the nonwoven cloth was supported thereon, thus preparing a reactant support.

The base agent and the reactant support obtained were combined to produce a material for formation of carbon dioxide external preparation.

### Example 17

### (Preparation of base agent)

Using the viscous material in Example 10, and a 0.4 mm-thick cotton facemask type nonwoven cloth as a polymeric three-dimensional network structure, 0.03 g of the aforementioned viscous material per square centimeter of the nonwoven cloth was impregnated therein, thus preparing a base agent.

### (Preparation of reactant support)

Using the reactant in Example 10, and as a support a 0.4 mm-thick cotton facemask type nonwoven cloth having a polyethylene film thereon as a backing, 0.03 g of the aforementioned reactant per square centimeter of the nonwoven cloth was supported thereon, thus preparing a reactant support supporting a reactant.

The base agent and the reactant support obtained were combined to produce a material for formation of carbon dioxide external preparation.

### Example 18

### (Preparation of base agent)

A base agent was prepared as in Example 11.

### (Preparation of reactant)

A reactant was prepared as in Example 15. -

The base agent and the reactant obtained were combined to produce a material for formation of carbon dioxide external preparation.

### Comparative Example

Manufacture was carried out as follows in accordance with Example 109 in Japanese Patent Application Laid-open No. 2000-319187.

### (Manufacture of acidic composition)

Using 25 wt% of citric acid, 25 wt% of ethyl cellulose and 50 wt% of croscarmellose sodium, porous columnar granules of length approximately 4 mm and diameter approximately 1 mm were manufactured using a wet extruding granulation method.

### (Manufacture of basic composition)

2.4 wt% of sodium hydrogen carbonate was dissolved in 89.6 wt% of purified water, and while gradually raising the temperature to 60°C, 4.0 wt% of sodium alginate, 2.0 wt% of ethyl cellulose and 2.0 wt% of sodium carboxymethyl cellulose were gradually added, and were dissolved in with stirring, and then the solution was left overnight, thus cooling the solution down to room temperature and hence manufacturing a viscous composition.

A composition for transdermal/transmucosal absorption of carbon dioxide, this being a composition containing carbon dioxide in the form of bubbles, was produced from the acidic composition and the basic composition obtained.

### Evaluation tests

Carbon dioxide external preparations obtained from the materials for formation of carbon dioxide external preparation in Examples, and the composition for transdermal/transmucosal absorption of carbon dioxide in Comparative Example manufactured as described above were evaluated as follows.

### Evaluation 1: Whitening and skin beautification effects for hands

For 18 female monitors, a carbon dioxide external preparation obtained from the material for formation of carbon dioxide external preparation in one of Examples 1 to 8 was applied for 5 minutes onto the back of the left hand and the composition for transdermal/transmucosal absorption of carbon dioxide in Comparative Example was applied for 5 minutes onto the back of the right hand, and the skin beautification effects were evaluated through the whiteness of the backs of the hands, the fineness of the grain of the skin, and the feel of the skin.

As the method of using the material for formation of carbon dioxide external preparation in Example 1, a 4 cm × 4 cm square piece of the base agent in Example 1 was patched onto the back of the left hand, and 1.2 g of the reactant in Example 1 was sprayed uniformly thereover.

As the method of using the material for formation of carbon dioxide external preparation in Example 2, a 4 cm × 4 cm square piece of the base agent in Example 2 was patched onto the back of the left hand, and 1.2 g of the reactant in Example 2 was applied uniformly thereover.

As the method of using the material for formation of carbon dioxide external preparation in Example 3, a 4 cm × 4 cm square piece of the base agent in Example 3 was patched onto the back of the left hand, and a 4 cm × 4 cm square piece of the reactant support in Example 3 was placed thereon.

As the method of using the material for formation of carbon dioxide external preparation in Example 4, a 4 cm × 4 cm square piece of the base agent in Example 4 was patched onto the back of the left hand, and a 4 cm × 4 cm square piece of the reactant support in Example 4 was placed thereon such that the polyethylene film was on the atmosphere side.

As the method of using the material for formation of carbon dioxide external preparation in Example 5, a 4 cm × 4 cm square piece of the base agent in Example 5 was patched onto the back of the left hand, and a 4 cm × 4 cm square piece of the reactant support in Example 5 was placed thereon such that the polyethylene film was on the atmosphere side.

As the method of using the material for formation of carbon dioxide external preparation in Example 6, a 4 cm × 4 cm square piece of the base agent in Example 6 was patched onto the back of the left hand, and 1.2 g of the reactant in Example 6 was applied uniformly thereover.

As the method of using the material for formation of carbon dioxide external preparation in Example 7, a 4 cm × 4 cm square piece of the base agent in Example 7 was patched onto the back of the left hand, and a 4 cm × 4 cm square piece of the reactant support in Example 7 was placed thereon such that the polyethylene film was on the atmosphere side.

As the method of using the material for formation of carbon dioxide external preparation in Example 8, a 4 cm × 4 cm square piece of the base agent in Example 8 was patched onto the back of the left hand, and 1.2 g of the reactant in Example 8 was applied uniformly thereover.

For the composition for transdermal/transmucosal absorption of carbon dioxide in Comparative Example, 1 g of the acidic composition was added to 20 g of the basic composition, stirring was carried out until the acidic composition dissolved completely, and 1.2 g of that was applied in a 4 cm × 4 cm square shape onto the back of the right hand of each of the 18 female monitors.

After 5 minutes, the carbon dioxide external preparations in Examples 1 to 8 and the composition for transdermal/transmucosal absorption of carbon dioxide in Comparative Example were completely removed from the backs of the hands, and the site of application of each of the preparations was inspected.

For the backs of the left hands on which the carbon dioxide external preparations in Examples 1 to 8 had been applied, immediately after the preparation was removed, in all of Examples, the skin had reddened in a 4 cm × 4 cm square shape matching the shape of application of the preparation, and the skin temperature had risen, and hence a very strong increase in blood flow was observed. The increase in blood flow was particularly strong in Example 4.

After a few minutes, the reddening disappeared in all of Examples, and then in all of Examples the skin turned white in a 4 cm × 4 cm square shape matching the shape of application of the carbon dioxide external preparation, and a feeling of moistness was obtained. The skin moisture content and the sebum content were measured using a facial analyzer (made by Corefront Corporation; measures the skin moisture content and the sebum content in a range of 0 to 99), whereupon the results were that for all of Examples the skin moisture content was in a range of 62 to 81 and the sebum content was in a range of 27 to 48 at the site on which the carbon dioxide external preparation in Examples had been applied, this being a clear increase compared with a skin moisture content in a range of 17 to 24 and a sebum content in a range of 0 to 11 for the surrounding skin, and correlating to the feeling of moistness. The skin surface was observed at a magnification of 40 × using a CCD camera, whereupon for all of Examples, at the site of application of the carbon dioxide external preparation, compared with the surrounding skin, the skin grooves and mounds were clearer, and the grain of the skin had become finer.

On the other hand, regarding the backs of the right hands on which the composition for transdermal/transmucosal absorption of carbon dioxide in Comparative Example had been applied, there were no monitors for which reddening was observed after the composition had been removed, and hence a blood flow increasing effect was not observed. Moreover, there were no monitors for which whitening and skin beautification effects were observed at the site of application; the skin moisture content increased slightly, but there was hardly any change in the sebum content.

### Evaluation 2: Whitening and skin beautification effects for hands

Using a total of 11 female monitors, one of the materials for formation of carbon dioxide external preparations in Examples 10 to 16 was applied for 5 minutes onto the back of the left hand and the composition for transdermal/transmucosal absorption of carbon dioxide in Comparative Example was applied for 5 minutes onto the back of the right hand, and the skin beautification effects were evaluated through the whiteness of the backs of the hands, the fineness of the grain of the skin, and the feel of the skin.

As the method of using the material for formation of carbon dioxide external preparation in each of Examples 10, 11, 14 and 15, a 5 cm × 5 cm square piece of the base agent was patched onto the back of the left hand, and 0.8 g of the corresponding reactant was applied uniformly thereover.

As the method of using the material for formation of carbon dioxide external preparation in each of Examples 12, 13 and 16, a 5 cm × 5 cm square piece of the base agent was patched onto the back of the left hand, and a 5 cm × 5 cm square piece of the corresponding reactant support was placed thereon.

For the composition for transdermal/transmucosal absorption of carbon dioxide in Comparative Example, 1 g of the acidic composition was added to 20 g of the basic composition, stirring was carried out until the acidic composition dissolved completely, and 1.5 g of that was applied in a 5 cm × 5 cm square shape onto the back of the right hand of each of the female monitors.

After 5 minutes, the carbon dioxide external preparations in Examples 10 to 16 and the composition for transdermal/transmucosal absorption of carbon dioxide in Comparative Example were completely removed from the backs of the hands, and the site of application of each of the preparations was inspected.

For the backs of the left hands on which the carbon dioxide external preparations in Examples 10 to 16 had been applied, immediately after the preparation was removed, for all of Examples, the skin had reddened in a 5 cm × 5 cm square shape matching the shape of application of the preparation, and the skin temperature had risen, and hence a very strong increase in blood flow was observed. The increase in blood flow was particularly strong in Examples 10 and 11.

After a few minutes, the reddening disappeared in all of Examples, and then in all of Examples the skin turned white in a 5 cm × 5 cm square shape matching the shape of application of the carbon dioxide external preparation, and a feeling of moistness was obtained. In particular, the whitening effect was most marked for the carbon dioxide external preparation in Example 16. The skin moisture content and the sebum content were measured using a facial analyzer (made by Corefront Corporation; measures the skin moisture content and the sebum content in a range of 0 to 99), whereupon the results were that for all of Examples 10 to 16 the skin moisture content was in a range of 62 to 87 and the sebum content was in a range of 27 to 62 at the site on which the carbon dioxide external preparation in Examples had been applied, this being a clear increase compared with a skin moisture content in a range of 17 to 24 and a sebum content in a range of 0 to 11 for the surrounding skin, and correlating to the feeling of moistness. The skin surface was observed at a magnification of 40x using a CCD camera, whereupon for all of Examples, at the site of application of the carbon dioxide external preparation, compared with the surrounding skin, the skin grooves and mounds were clearer, and the grain of the skin had become finer.

On the other hand, regarding the backs of the right hands on which the composition for transdermal/transmucosal absorption of carbon dioxide in Comparative Example had been applied, there were no monitors for which reddening was observed after the composition had been removed, and hence a blood flow increasing effect was not observed. Moreover, there were no monitors for which whitening and skin beautification effects were observed at the site of application; the skin moisture content increased slightly, but there was hardly any change in the sebum content.

### Evaluation 3: Partial slimming, whitening and skin beautification effects for face (1)

The base agent using a facemask type nonwoven cloth in Example 9 was patched onto the face of a 26-year-old female monitor, and the reactant support using a facemask type nonwoven cloth in Example 9 was placed thereon such that the polyethylene film was on the atmosphere side and patched on for 10 minutes. Approximately 1 minute after patching on, the monitor said that the whole of the skin on her face was hot, and the skin under the aforementioned external preparation had turned red, and hence promotion of the blood flow was observed. After the aforementioned external preparation was removed, all of the skin was red, and the skin temperature was raised, but after 1 to 2 minutes, the redness went away and the skin temperature returned to normal, and moreover the skin turned very white, and appeared clear. Moreover, the cheeks had become smaller, and hence a face slimming effect was observed.

### Evaluation 4: Partial slimming, whitening and skin beautification effects for face (2)

The base agent using a facemask type nonwoven cloth in Example 9 cut vertically in half was patched onto the right half of the face of a 26-year-old female, and the reactant support using a facemask type nonwoven cloth in Example 9 cut vertically in half was placed thereon such that the polyethylene film was on the atmosphere side and patched on for 5 minutes. 15 g of the carbon dioxide external preparation in Comparative Example was similarly applied to a uniform thickness onto the left half of the face for 5 minutes. For the right half of the face on which the carbon dioxide external preparation in Example 9 had been patched, approximately 1 minute after patching on, the woman said that the skin was hot, and hence an increase in blood flow was observed, whereas the left half of the face on which the carbon dioxide external preparation in Comparative Example had been applied felt cold. After 5 minutes, the preparations were removed, whereupon for the right half of the face, the whole thereof was red, and the skin temperature was raised, but after approximately 2 minutes, the redness went away and the skin temperature returned to normal. On the other hand, the left half of the face remained cold, and there was no redness or the like, and hence an increase in blood flow was not observed. Comparing the left and right of the face, for the right half of the face the skin was whiter, and appeared clearer, and hence whitening and skin beautification effects were observed. Moreover, the right cheek had become smaller than the left cheek and the corner of the mouth was raised, and hence a partial slimming effect of the face was observed.

### Evaluation 5: Partial slimming, whitening and skin beautification effects for face (3)

The base agent using a facemask type nonwoven cloth in Example 17 was patched onto the face of each of a 37-year-old female monitor and a 40-year-old female monitor, and the reactant support using a facemask type nonwoven cloth in Example 17 was placed thereon such that the polyethylene filmwas on the atmosphere side and patched on for 10 minutes. Approximately 1 minute after patching on, both monitors said that the whole of the skin on her face was hot, and the skin under the external preparation had turned red, and hence promotion of the blood flow was observed. After the aforementioned external preparation was removed, all of the skin was red on both monitors, and the skin temperature was raised, but after 1 to 2 minutes, the redness went away and the skin temperature returned to normal, and moreover the skin turned very white, and appeared clear. Moreover, the cheeks had become smaller, and hence a face slimming effect was observed.

### Evaluation 6: Partial slimming, whitening and skin beautification effects for face (4)

The base agent using a facemask type nonwoven cloth in Example 17 cut vertically in half was patched onto the right half of the face of each of a 37-year-old female monitor and a 40-year-old female monitor, and the reactant support using a facemask type nonwoven cloth in Example 17 cut vertically in half was placed thereon such that the polyethylene film was on the atmosphere side and patched on for 5 minutes. 15 g of the carbon dioxide external preparation in Comparative Example was similarly applied to a uniform thickness onto the left half of the face for 5 minutes. For the right half of the face on which the carbon dioxide external preparation in Example 17 had been patched, approximately 1 minute after patching on, both monitors said that the skin was hot, and hence an increase in blood flow was observed, whereas the left half of the face on which the carbon dioxide external preparation in Comparative Example had been applied felt cold for both monitors. After 5 minutes, the preparations were removed, whereupon for both monitors, for the right half of the face, the whole thereof was red, and the skin temperature was raised, but after approximately 2 minutes, the redness went away and the skin temperature returned to normal. On the other hand, for both monitors, the left half of the face remained cold, and there was no redness or the like, and hence an increase in blood flow was not observed. Comparing the left and right of the face, for both monitors, for the right half of the face the skin was whiter, and appeared clearer, and hence whitening and skin beautification effects were observed. Moreover, the right cheek had become smaller than the left cheek and the corner of the mouth was raised, and hence a partial slimming effect of the face was observed.

### Evaluation 7: Effect on cut

A 1 cm × 1 cm square piece of the base agent in Example 12 was patched onto a cut of length 7 mm on the left hand ring finger of a 28-year-old female, and a 1 cm × 1 cm square piece of the reactant support in Example 12 was placed thereon. The pain of the wound went away immediately, and when the preparation was peeled off after 15 minutes, the wound had closed up.

### Evaluation 8: Effect on graze

A 4 cm × 4 cm square piece of the base agent in Example 6 was patched onto a graze of size 2 cm × 3 cm on the right elbow of a 46-year-oldmale, 1.2 g of the hydrogel-like reactant in Example 6 was applied uniformly thereover, and then this was covered with a 4.4 cm × 4.4 cm synthetic polymer wound-covering material (trade name Tegaderm, made by 3M Health Care). The carbon dioxide external preparation in Example 6 was applied once per day in this way, being exchanged every day, whereupon after the fourth application, the wound had completely closed up and healed.

### Evaluation 9: Effect on atopic dermatitis (1)

An 8 cm × 8 cm square piece of the base agent in Example 3 was patched onto atopic dermatitis accompanied by bleeding and scabs on the backs of both hands of a 23-year-old male, and an 8 cm × 8 cm square piece of the reactant support in Example 3 was placed thereon for 10 minutes. This was carried out once per day, whereupon by the second day the bleeding had all stopped, and by the seventh day the scabs had disappeared and had healed.

### Evaluation 10: Effect on atopic dermatitis (2)

An 8 cm × 8 cm square piece of the base agent in Example 13 was patched onto atopic dermatitis accompanied by bleeding and scabs on the backs of both hands of a 32-year-old male, and an 8 cm × 8 cm square piece of the reactant support in Example 13 was placed thereon for 10 minutes. Upon removing the carbon dioxide external preparation in Example 13, some of the scabs came off and the bleeding had virtually stopped. This was carried out once per day, whereupon by the eighth day the scabs had disappeared and had healed.

### Evaluation 11: Effect on pimples

A 4 cm × 14 cm piece of the base agent in Example 18 was patched onto a large number of pimples accompanied by redness on the forehead of a 29-year-old female, and a piece of the reactant support in Example 18 of the same size was placed thereon for 10 minutes. This was carried out once per day, whereupon the redness of the pimples became fainter, and there was also an improvement in the roughness of the skin as a whole. Upon carrying this out once per day, by the eleventh day the pimples had completely disappeared, and the skin had become white and smooth.

### INDUSTRIAL APPLICABILITY

As described above, a material for formation of carbon dioxide external preparation of the present invention can be easily used, and moreover with the carbon dioxide external preparation obtained from such a material for formation of carbon dioxide external preparation, the carbon dioxide does not form bubbles and hence does not diffuse into the atmosphere, and thus can be transdermally or transmucosally absorbed efficiently, and hence strong cosmetic and medical effects are obtained in a short time. The material for formation of carbon dioxide external preparation and carbon dioxide external preparation can thus be suitably used as cosmetics, medical external preparations, and so on.

## Claims

1. A material for formation of carbon dioxide external preparation, comprising:
- a base agent that comprises an elastic polymeric three-dimensional network structure impregnated with a viscous material containing at least an acid and water, and is made to contact with the skin in use; and
- a reactant that contains at least a carbonate, and is made to contact with the base agent in use so as to generate carbon dioxide,
- wherein the generated carbon dioxide immediately dissolves in the viscous material in a non-bubble state.

2. The material according to claim 1,
wherein the elastic polymeric three-dimensional network structure is sheet-like.

3. The material according to claim 1,
wherein the elastic polymeric three-dimensional network structure is a fibrous or porous absorbent.

4. The material according to claim 1,
wherein the viscous material contains at least one thickener selected from the group consisting of sodium alginate, propylene glycol alginate ester, carrageenan, carboxyvinyl polymers, polyvinyl alcohol and polyvinyl pyrrolidone.

5. The material according to claim 1,
wherein the viscous material is an emulsion or cream containing at least an acid, water, an oil and a surfactant.

6. The material according to claim 1,
wherein the viscous material contains at least one thickener selected from the group consisting of sodium alginate, propylene glycol alginate ester, carrageenan, carboxyvinyl polymers, polyvinyl alcohol and polyvinyl pyrrolidone,
wherein the acid in the viscous material is at least one selected from the group consisting of malic acid, succinic acid, sodium dihydrogen phosphate, carboxyvinyl polymers, citric acid, L-ascorbic acid and tartaric acid, and wherein the elastic polymeric three-dimensional network structure is a nonwoven cloth or a sponge.

7. The material according to claim 1,
wherein the reactant is a viscous material further containing a thickener and water.

8. The material according to claim 1,
wherein the reactant contains water and at least one thickener selected from the group consisting of sodium alginate, propylene glycol alginate ester, carrageenan, polyvinyl alcohol and polyvinyl pyrrolidone,
and the carbonate is sodium hydrogencarbonate.

9. The material according to claim 1,
wherein the viscous material in the base agent contains at least one thickener selected from the group consisting of sodium alginate, propylene glycol alginate ester, carrageenan, carboxyvinyl polymers, polyvinyl alcohol and polyvinyl pyrrolidone,
wherein the acid in the viscous material is at least one selected from the group consisting of malic acid, succinic acid, sodium dihydrogen phosphate, carboxyvinyl polymers, citric acid, L-ascorbic acid and tartaric acid, wherein the elastic polymeric three-dimensional network structure is a nonwoven cloth or a sponge,
wherein the reactant contains water and at least one thickener selected from the group consisting of sodium alginate, propylene glycol alginate ester, carrageenan, polyvinyl alcohol and polyvinyl pyrrolidone,
and the carbonate is sodium hydrogencarbonate.

10. The material according to claim 1,
wherein the reactant is supported on a sheet-like or bag-shaped support.

11. The material according to claim 10,
wherein the support on which the reactant is supported, the surface that is on the atmosphere side in use is covered with a covering material that is impermeable to or has low permeability to carbon dioxide.

## Patentansprüche

1. Material zum Herstellen eines äußerlichen Kohlendioxidpräparats,
das folgendes aufweist:
- ein basisches Mittel, das eine elastische, polymere, dreidimensionale Netzwerkstruktur aufweist, die mit einem viskosen Material imprägniert ist, das zumindest eine Säure und Wasser enthält, und das bei Verwendung für den Hautkontakt bestimmt ist; und
- einen Reaktanten, der zumindest ein Carbonat enthält und bei Verwendung für den Kontakt mit dem basischen Mittel bestimmt ist, so daß Kohlendioxid erzeugt wird;
- wobei sich das erzeugte Kohlendioxid sofort in einem blasenfreien Zustand in dem viskosen Material löst.

2. Material nach Anspruch 1,
wobei die elastische, polymere, dreidimensionale Netzwerkstruktur flächengebildeartig ist.

3. Material nach Anspruch 1,
wobei die elastische, polymere, dreidimensionale Netzwerkstruktur ein faserförmiges oder poröses Absorptionsmittel ist.

4. Material nach Anspruch 1,
wobei das viskose Material zumindest ein Verdickungsmittel enthält, das aus der Gruppe ausgewählt ist, die aus Natriumalginat, Propylenglycolalginatester, Carrageen, Carboxyvinylpolymeren, Polyvinylalkohol und Polyvinylpyrrolidon besteht.

5. Material nach Anspruch 1,
wobei das viskose Material eine Emulsion oder Creme ist, die zumindest eine Säure, Wasser, ein Öl und ein oberflächenaktives Mittel enthält.

6. Material nach Anspruch 1,
wobei das viskose Material zumindest ein Verdickungsmittel enthält, das aus der Gruppe ausgewählt ist, die aus Natriumalginat, Propylenglycolalginatester, Carrageen, Carboxyvinylpolymeren, Polyvinylalkohol und Polyvinylpyrrolidon besteht,
wobei die Säure in dem viskosen Material zumindest eine solche ist, die aus der Gruppe ausgewählt ist, die aus Äpfelsäure, Succinsäure, Natriumdihydrogenphosphat, Carboxyvinylpolymeren, Citronensäure, L-Ascorbinsäure und Weinsäure besteht, und
wobei die elastische, polymere, dreidimensionale Netzwerkstruktur ein ungewebter Stoff oder ein Schwamm ist.

7. Material nach Anspruch 1,
wobei der Reaktant ein viskoses Material ist, das ferner ein Verdickungsmittel und Wasser enthält.

8. Material nach Anspruch 1,
wobei der Reaktant Wasser und zumindest ein Verdickungsmittel enthält, das aus der Gruppe ausgewählt ist, die aus Natriumalginat, Propylenglycolalginatester, Carrageen, Polyvinylalkohol und Polyvinylpyrrolidon besteht, und
wobei das Carbonat Natriumhydrogencarbonat ist.

9. Material nach Anspruch 1,
wobei das viskose Material im basischen Mittel zumindest ein Verdickungsmittel enthält, das aus der Gruppe ausgewählt ist, die aus Natriumalginat, Propylenglycolalginatester, Carrageen, Carboxyvinylpolymeren, Polyvinylalkohol und Polyvinylpyrrolidon besteht,
wobei die Säure im viskosen Material zumindest eine solche ist, die aus der Gruppe ausgewählt ist, die aus Äpfelsäure, Succinsäure, Natriumdihydrogenphosphat, Carboxyvinylpolymeren, Citronensäure, L-Ascorbinsäure und Weinsäure besteht, und
wobei die elastische, polymere, dreidimensionale Netzwerkstruktur ein ungewebter Stoff oder ein Schwamm ist,
wobei der Reaktant Wasser und zumindest ein Verdickungsmittel enthält, das aus der Gruppe ausgewählt ist, die aus Natriumalginat, Propylenglycolalginatester, Carrageen, Polyvinylalkohol und Polyvinylpyrrolidon besteht, und
wobei das Carbonat Natriumhydrogencarbonat ist.

10. Material nach Anspruch 1,
wobei der Reaktant von einem flächengebildeartigen oder beutelförmigen Träger getragen ist.

11. Material nach Anspruch 10,
wobei bei dem Träger, von dem der Reaktant getragen wird, die Oberfläche, die sich bei Verwendung auf der Atmosphärenseite befindet, mit einem Abdeckmaterial bedeckt ist, das für Kohlendioxid undurchlässig oder wenig durchlässig ist.

## Revendications

1. Matériau pour la formation d'une préparation externe de dioxyde de carbone comprenant:
- un agent de base qui comprend une structure de réseau tridimensionnel polymère élastique imprégnée avec un matériau visqueux contenant au moins un acide et de l'eau, et mise en contact avec la peau lors de l'utilisation; et
- un corps réagissant qui contient au moins un carbonate et qui est mis en contact avec l'agent de base lors de l'utilisation afin de produire du dioxyde de carbone,
- dans lequel le dioxyde de carbone produit se dissout immédiatement dans le matériau visqueux dans un état de non-bulle.

2. Matériau selon la revendication 1,
dans lequel la structure de réseau tridimensionnel polymère élastique est dans la forme d'une feuille.

3. Matériau selon la revendication 1,
dans lequel la structure de réseau tridimensionnel polymère élastique est un absorbant fibreux ou poreux.

4. Matériau selon la revendication 1,
dans lequel le matériau visqueux contient au moins un épaississant choisi dans le groupe constitué de l'alginate de sodium, d'un ester d'alginate de propylèneglycol, du carrageenan, de polymères carboxyvinyliques, de poly(alcool vinylique) et de polyvinylpyrrolidone.

5. Matériau selon la revendication 1,
dans lequel le matériau visqueux est une émulsion ou une crème contenant au moins un acide, de l'eau, une huile et un tensioactif.

6. Matériau selon la revendication 1,
dans lequel le matériau visqueux contient au moins un épaississant choisi dans le groupe constitué de l'alginate de sodium, d'un ester d'alginate de propylèneglycol, du carrageenan, de polymères carboxyvinyliques, de poly(alcool vinylique) et de polyvinylpyrrolidone,
dans lequel l'acide dans le matériau visqueux est au moins un choisi dans le groupe constitué de l'acide malique, de l'acide succinique, dudit hydrogénophosphate de sodium, de polymères carboxyvinyliques, d'acide citrique, d'acide L-ascorbique et d'acide tartarique, et dans lequel la structure de réseau tridimensionnel polymère élastique est un non-tissé ou une éponge.

7. Matériau selon la revendication 1,
dans lequel le corps réagissant est un matériau visqueux contenant en plus un épaississant et de l'eau.

8. Matériau selon la revendication 1,
dans lequel le corps réagissant contient de l'eau et au moins un épaississant choisi dans le groupe constitué de l'alginate de sodium, d'un ester d'alginate de propylèneglycol, du carrageenan,
du poly(alcool vinylique) et de la polyvinylpyrrolidone, et dans lequel le carbonate est d'hydrogénocarbonate de sodium.

9. Matériau selon la revendication 1,
dans lequel le matériau visqueux dans l'agent de base contient au moins un épaississant choisi dans le groupe constitué de l'alginate de sodium, d'un ester d'alginate de propylèneglycol, du carrageenan, de polymères carboxyvinyliques, de poly(alcool vinylique) et de polyvinylpyrrolidone,
dans lequel l'acide dans le matériau visqueux est au moins un choisi dans le groupe constitué de l'acide malique, de l'acide succinique, du dihydrogénophosphate de sodium, de polymères carboxyvinyliques, d'acide citrique, d'acide L-ascorbique et d'acide tartarique, dans lequel la structure de réseau tridimensionnel polymère élastique est un non-tissé ou une éponge,
dans lequel le corps réagissant contient de l'eau et au moins un épaississant choisi dans le groupe constitué de l'alginate de sodium, d'un ester d'alginate de propylèneglycol, de carrageenan, de poly(alcool vinylique) et de polyvinylpyrrolidone, et
dans lequel le carbonate et d'hydrogénocarbonate de sodium.

10. Matériau selon la revendication 1,
dans lequel le corps réagissant est supporté sur un support en forme de feuille ou en forme de sac.

11. Matériau selon la revendication 10,
dans lequel le support sur lequel est supporté le corps réagissant, la surface qui est sur le côté de l'atmosphère lors de l'utilisation est recouverte avec un matériau de couverture qui est imperméable audit oxyde de carbone ou qui présente une faible perméabilité au dioxyde de carbone.
